(19) Europäisches Patentamt
European Patent Office
Office européen des brevets

(11) EP 0 955 298 B1

(12) # FASCICULE DE BREVET EUROPEEN

(45) Date de publication et mention de la délivrance du brevet:
**14.11.2001 Bulletin 2001/46**

(51) Int Cl.$^7$: **C07D 317/36**

(21) Numéro de dépôt: **99390009.1**

(22) Date de dépôt: **22.04.1999**

(54) **Procédé de fabrication de carbonate de glycérol**

Verfahren zur Herstellung von Glycerincarbonate

Process for the preparation of glycerol carbonate

(84) Etats contractants désignés:
**BE DE FR GB NL**

(30) Priorité: **30.04.1998 FR 9805547**

(43) Date de publication de la demande:
**10.11.1999 Bulletin 1999/45**

(73) Titulaire: **ORGANISATION NATIONALE INTERPROFESSIONNELLE DES OLEAGINEUX-ONIDOL**
**75008 Paris (FR)**

(72) Inventeurs:
- **Claude, Sylvain**
**75014 Paris (FR)**
- **Mouloungui, Zéphirin**
**31500 Toulouse (FR)**
- **Yoo, Jeong-Woo**
**31500 Toulouse (FR)**
- **Gaset, Antoine**
**31000 Toulouse (FR)**

(74) Mandataire:
**Cabinet BARRE LAFORGUE & associés**
**95, rue des Amidonniers**
**31000 Toulouse (FR)**

(56) Documents cités:
**EP-A- 0 581 131** **FR-A- 2 733 232**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 955 298 B1

## Description

L'invention concerne un procédé de fabrication de carbonate de glycérol :

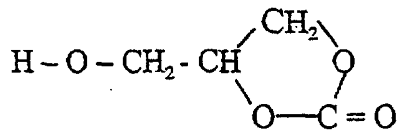

Le carbonate de glycérol est un composé bi-fonctionnel et sa double fonctionnalité lui permet de jouer le rôle de solvant à l'égard de nombreux composés organiques ou minéraux (domaine cosmétique, peintures, accumulateurs...). Ce solvant polaire protique est recherché en raison de sa non-toxicité et de son haut point d'ébullition. Le carbonate de glycérol peut également servir d'additif pour la stabilisation de polymères, de monomères de polymérisation pour la synthèse des polycarbonates, et d'intermédiaires de synthèses dans certaines réactions organiques (estérification, transestérification, carbamoylation...).

Toutefois, ce composé n'est actuellement pas fabriqué sur le plan industriel ; il est obtenu en laboratoire par réaction du glycérol avec un carbonate organique (brevets US 2915529, FR 2733232). Le défaut essentiel de cette réaction de carbonatation du glycérol réside dans le coût du réactif utilisé comme source de carbonate.

L'invention se propose de fournir un nouveau procédé de fabrication de carbonate de glycérol par une réaction catalytique de carbamoylation/carbonatation du glycérol utilisant un réactif beaucoup moins onéreux que les carbonates organiques.

Un autre objectif est d'obtenir le carbonate de glycérol dans un état de grande pureté sans qu'il soit nécessaire de le séparer des coproduits.

Un autre objectif est de permettre l'obtention du carbonate de glycérol à partir de réactifs de départ impurs, en particulier de composés bruts disponibles dans le commerce.

A cet effet, le procédé conforme à l'invention en vue de fabriquer du carbonate de glycérol consiste à faire réagir de l'urée et du glycérol à une température comprise entre 90° C et 220° C en présence d'un catalyseur portant des sites acides de Lewis associés à un ou des contre-ions anioniques d'hétéro-atome (c'est-à-dire non carboné).

Le catalyseur peut en particulier être constitué par un sel métallique ou organométallique, ou un mélange de sels métalliques ou organométalliques, se présentant sous forme de poudre solide. On peut également utiliser un composé métallique supporté, sous forme de solide macroporeux ou de gel, comprenant une matrice polymérique organique supportant sur des groupements fonctionnels hétéro-organiques des cations métalliques dotés des sites acides de Lewis.

Ainsi, le procédé de l'invention utilise comme réactif de départ l'urée qui est un composé peu onéreux, disponible en grande quantité. Les expérimentations ont permis de constater que ce composé réagissait à chaud avec le glycérol en présence d'un catalyseur du type précité selon le mécanisme suivant :

(1) urée + glycérol $\rightarrow$ carbamate de glycérol + ammoniac

(2) carbamate de glycérol $\rightarrow$ carbonate de glycérol + ammoniac

La deuxième étape présente une cinétique plus lente que la première et la présence du catalyseur précité évite que cette étape soit bloquante. De préférence, la réaction est réalisée sous vide, en particulier à une pression comprise entre $10^3$ et $2.10^4$ pascals, de façon à déplacer l'équilibre des réactions par élimination de l'ammoniac à l'état gazeux.

Les expérimentations ont montré que le glycérol pouvait être apporté sous forme de glycérine dite technique, issue de réactions lipochimiques de base (alcoolyse, hydrolyse, saponification des huiles ou graisses animales ou végétales).

On a montré que les sulfates métalliques ou sulfates organométalliques (ou mélange de sulfates) donnaient une très bonne efficacité catalytique, le cation métallique étant de préférence choisi de façon que le sel présente une solubilité à froid dans l'eau supérieure à 5 g par litre. On a montré également qu'une calcination préalable de ce type de catalyseur est avantageuse pour accroître son activité catalytique (cette calcination réduit le nombre de sites de Brônsted). En particulier, on peut utiliser comme sel un sulfate métallique (ou un mélange de sulfates) du groupe suivant : sulfate de manganèse, sulfate de zinc, sulfate de magnésium, sulfate de nickel, sulfate de fer, sulfate de cobalt. On peut également utiliser un sulfate organométallique de zinc. Ces sulfates solides présentent des sites acides

de Lewis, nombreux et de force élevée (en particulier après calcination), sites qui sont responsables de l'essentiel de l'activité catalytique engendrant une activation très efficace de l'étape de carbonatation (2). Le catalyseur métallique du type ci-dessus évoqué est de préférence ajouté dans une proportion pondérale (par rapport au glycérol) comprise entre 1 % et 8 %, la valeur choisie dans cette plage étant fonction du nombre et de la force des sites de Lewis que contient le sel utilisé.

Comme déjà évoqué, on peut également utiliser comme catalyseur un composé métallique supporté comprenant une matrice polymérique, en particulier polystyrénique, supportant les cations métalliques, en particulier $Zn^{++}$, $Mg^{++}$, $Mn^{++}$, $Fe^{++}$, $Ni^{++}$, $Cd^{++}$ ; associés à des groupements fonctionnels hétéro-organiques, en particulier sulfonates. Un tel composé métallique supporté peut avantageusement être fabriqué par échange d'ions à partir d'une matrice polymérique sulfonique disponible sur le marché ; l'échange est réalisé de façon à fixer sur les groupements sulfonates de ladite matrice les cations métalliques dotés des sites acides de Lewis. L'addition du composé métallique supporté du type précité est de préférence effectuée de façon que la quantité de cations métalliques par mole de glycérol soit comprise entre 10 et 100 milliéquivalent gramme.

Dans le cas où un tel composé métallique supporté est utilisé, il est souhaitable que le milieu réactionnel soit anhydre pour écarter tout risque de dégradation du catalyseur. Selon un mode préférentiel de mise en oeuvre, le glycérol et le catalyseur sont d'abord mis en présence à la pression réduite précitée avec évacuation de la vapeur d'eau dégagée, l'urée étant ensuite ajoutée après élimination de l'eau libre du milieu réactionnel.

Par ailleurs, on ajuste avantageusement les quantités d'urée et de glycérol mises en présence, de sorte que les proportions molaires relatives de ces composés soient comprises entre 0,65 et 1,20 mole d'urée par mole de glycérol. On obtient ainsi un couplage optimum entre les deux étapes : carbamoylation (1) et carbonatation (2).

Les réactions de carbamoylation (1) et de carbonatation (2) mises en oeuvre dans le procédé de l'invention n'ont jamais été utilisées avec le glycérol pour l'obtention du carbonate de glycérol. Il est à noter que des réactions de carbonatation faisant intervenir l'urée ont déjà été décrites à partir soit d'un monoalcool ("Shaikh et Sivaram, Organic Carbonates, Chem. Rev. 1996, 96, p 951-976" ; "Ball et al, Synthesis of carbonates and polycarbonates by reaction of urea with hydroxy compounds, Mol. Chem. 1984, vol. 1, p 95-108") soit d'un diol (EP 0443758 ; EP 0581131); toutefois, les catalyseurs préconisés pour ces réactions effectuées avec un monoalcool ou un diol sont, pour la grande majorité, inefficaces dans le cas du glycérol (et plus généralement dans le cas des triols) comme le montrent les essais comparatifs E-H fournis plus loin.

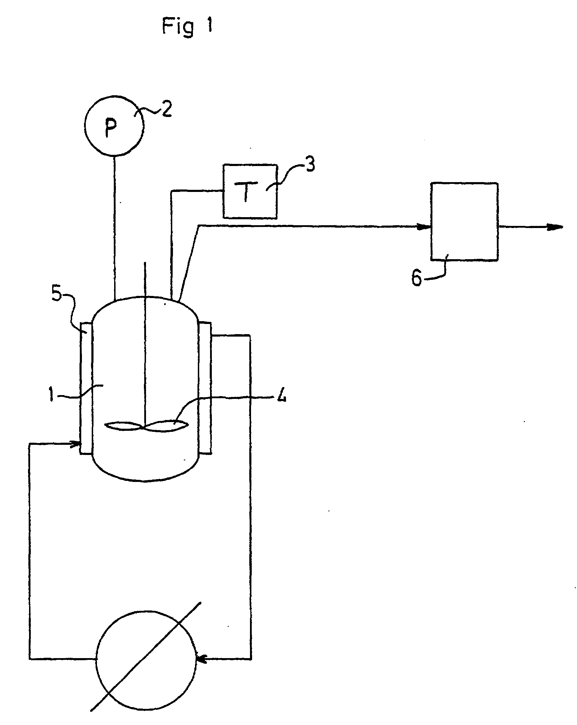

Les exemples 1 à 21 et les essais comparatifs A à H qui suivent ont été mis en oeuvre dans une installation à fonctionnement discontinu telle que représentée à la figure 1.

Cette installation comprend un réacteur semi-fermé 1 (250 ml) muni d'un capteur de pression 2, d'un capteur de température 3, d'un agitateur mécanique 4, d'une double enceinte 5 contenant un bain d'huile chauffant, et d'une pompe à vide 6 pour l'extraction des vapeurs et de l'ammoniac, permettant d'ajuster la pression dans le réacteur à une valeur d'environ 40 mbar (environ $4.10^3$ pascals).

Sauf indication contraire, les rendements fournis dans les exemples et essais comparatifs sont des rendements molaires en carbonate de glycérol rapporté au glycérol de départ.

EXEMPLE 1

Dans le réacteur 1 de la figure 1, on mélange 27,6 g (0,3 mole) de glycérol, 18,0 g (0,3 mole) d'urée et 1,7 g de sulfate de manganèse calciné à 450° C pendant 3 heures (rapport molaire urée/glycérol = 1 ; proportion pondérale catalyseur/glycérol = 0,086). On porte le mélange réactionnel à 150° C et on abaisse la pression à 40 mbar sous agitation mécanique pendant 2 heures. Pendant la réaction, l'ammoniac formé est éliminé par la pompe à vide 6. Après 2 heures, le mélange réactionnel brut est analysé par chromatographie en phase gazeuse sur colonne capillaire "Carbowax 20 M" (12 m) avec le tétraéthylène glycol comme étalon interne. 16,8 g (0,183 mole) de glycérol sont transformés en 21, 6 g du carbonate de glycérol (soit un rendement molaire de 61 % par rapport au glycérol).

EXEMPLE 2

La mise en oeuvre est identique à celle de l'exemple 1, mais on diminue la quantité de catalyseur et on augmente la durée de la réaction. On utilise 0,5 g de sulfate de manganèse calciné à 450° C (proportion pondérale catalyseur/glycérol = 0,027). On réalise la réaction à 150° C et 40 mbar pendant 6 heures. Après 6 heures, le mélange réactionnel brut est analysé par chromatographie en phase gazeuse sur colonne capillaire "Carbowax 20 M" (12 m) avec le tétraéthylène glycol comme étalon interne. 20,2 g (0,22 mole) de glycérol sont transformés en 26,0 g du carbonate de glycérol (soit un rendement molaire de 73 % par rapport au glycérol).

EXEMPLES 3 - 4

Dans le réacteur 1 de la figure 1, on mélange 27,6 g (0,3 mole) de glycérol, 18,0 g (0,3 mole) d'urée et 1,7 g de sulfate de magnésium calciné à 375° C pendant 3 heures. On porte le mélange réactionnel à 150° C et on abaisse la pression à 40 mbar sous agitation mécanique pendant 2 heures (exemple 3) et 5 heures (exemple 4). Pendant la réaction, l'ammoniac formé est éliminé par la pompe à vide 6. Après la réaction, le mélange réactionnel brut est analysé par chromatographie en phase gazeuse sur colonne capillaire "Carbowax 20 M" (12 m) avec le tétraéthylène glycol comme étalon interne. Les rendements molaires par rapport au glycérol sont indiqués dans le tableau suivant :

| Exemple | Durée de réaction (h) | Rendement (%) |
|---|---|---|
| 3 | 2 | 44 |
| 4 | 5 | 58 |

EXEMPLE 5

La mise en oeuvre de cet exemple est identique à celle de l'exemple 4, mais on change le rapport molaire urée/glycérol. On mélange 27,6 g (0,3 mole) de glycérol, 12,0 g (0,2 mole) d'urée et 1,7 g de sulfate de magnésium calciné à 375° C pendant 3 heures (rapport molaire urée/glycérol = 0,67). On réalise la réaction à 150° C et 40 mbar pendant 5 heures. Après 5 heures, le mélange réactionnel brut est analysé par chromatographie en phase gazeuse sur colonne capillaire "Carbowax 20 M" (12 m) avec le tétraéthylène glycol comme étalon interne. 14,7 g (0,16 mole) de glycérol sont transformés en 18,9 g du carbonate de glycérol (soit un rendement molaire de 81 % par rapport à l'urée, et un rendement molaire de 54 % par rapport au glycérol).

EXEMPLES 6 - 7

Dans le réacteur 1 de la figure 1, on mélange 27,6 g (0,3 mole) de glycérol, 18,0 g (0,3 mole) d'urée et 1,5 g de sulfate de cobalt calciné à 450° C pendant 3 heures (rapport pondéral catalyseur/glycérol = 0,077). On porte le mélange réactionnel à 150° C et on abaisse la pression à 40 mbar sous agitation mécanique pendant 2 heures (exemple 6) et 5 heures (exemple 7). Pendant la réaction, l'ammoniac formé est éliminé par la pompe à vide 6. Après la réaction, le mélange réactionnel brut est analysé par chromatographie en phase gazeuse sur colonne capillaire "Carbowax 20 M" (12 m) avec le tétraéthylène glycol comme étalon interne. Les rendements molaires par rapport au glycérol sont indiqués dans le tableau suivant :

| Exemple | Durée de réaction (h) | Rendement (%) |
|---|---|---|
| 6 | 2 | 51 |
| 7 | 5 | 63 |

EXEMPLES 8 - 10

La mise en oeuvre dans ces exemples est identique à celle de l'exemple 1 en utilisant d'autres catalyseurs. Les catalyseurs utilisés et les rendements molaires par rapport au glycérol sont indiqués dans le tableau suivant, après 2 heures de réaction :

| Exemple | Catalyseur | Rendement | Remarque |
|---|---|---|---|
| 8 | $FeSO_4$ | 41 | calciné à 400° C |
| 9 | $FeSO_4.7H_2O$ | 37 | non calciné |
| 10 | $ZnSO_4$ | 80 | calciné à 550° C |

EXEMPLE 11

Dans le réacteur 1 de la figure 1, on mélange 27,6 g (0,3 mole) de glycérol, 18,0 g (0,3 mole) d'urée et 1,7 g de sulfate de zinc. On porte le mélange réactionnel à 130°C et on abaisse la pression à 40 mbar sous agitation mécanique pendant 5 heures. Pendant la réaction, l'ammoniac formé est éliminé par la pompe à vide 6. Après 5 heures, le mélange réactionnel brut est analysé par chromatographie en phase gazeuse sur colonne capillaire "Carbowax 20

M" (12 m) avec le tétraéthylène glycol comme étalon interne. 22,9 g (0,25 mole) de glycérol sont transformés en 29,4 g du carbonate de glycérol (soit un rendement molaire de 83 % par rapport au glycérol).

Essai comparatif de référence A (absence de catalyseur)

On reproduit la mise en oeuvre de l'exemple 1 mais en l'absence de catalyseur, de façon à permettre une comparaison des rendements obtenus en carbonate de glycérol.
On obtient un rendement molaire par rapport au glycérol de 26 %.

Essais comparatifs B, C, D

La mise en oeuvre de ces essais comparatifs est identique à celle de l'exemple 1 en utilisant des catalyseurs insolubles (solubilité à froid dans l'eau inférieure à 5 g/l), ayant peu de sites acides de Lewis. Ces essais comparés aux exemples qui précèdent montrent l'efficacité des sites de Lewis dans la catalyse. A noter que, pour l'essai D, le sulfate de strontium a été calciné mais que ce traitement n'augmente pas de façon très significative le rendement en carbonate. Dans l'essai B, il semble que le sulfate de strontium insoluble ait plutôt un effet défavorable (comparé à l'essai de référence A), probablement en raison du nombre de ses sites de Brönsted. Les catalyseurs utilisés et les rendements molaires par rapport au glycérol sont indiqués dans le tableau suivant, après 2 heures de réaction :

| N° de l'essai | Catalyseur | Rendement (%) | Remarque |
|---|---|---|---|
| B | $SrSO_4$ | 25 | non calciné |
| C | $BaSO_4$ | 28 | non calciné |
| D | $SrSO_4$ | 27 | calciné à 400° C |

Essais comparatifs E - H

On reproduit la mise en oeuvre de l'exemple 1 et de l'essai de référence A en utilisant certains catalyseurs préconisés dans l'art antérieur pour des réactions de carbonatation faisant intervenir l'urée. Les catalyseurs utilisés et les rendements molaires par rapport au glycérol sont indiqués dans le tableau suivant:

| Essai | Catalyseur | Rendement (%) | Remarque : catalyseur préconisé dans : |
|---|---|---|---|
| E | Oxyde de dibutyl étain (1 g) | 27 | EP 0443758 |
| F | Oxyde de magnésium (1 g) | 32 | EP 0581131 |
| G | Acétate de plomb (1 g) | 28 | EP 0581131 |
| H | Chlorure de calcium (1 g) | 25 | EP 0581131 |

L'efficacité de ces catalyseurs sur la réaction de l'urée avec le glycérol est très faible, voire nulle.

EXEMPLE 12

Dans le réacteur 1 de la figure 1, on mélange 27,6 g (0,3 mole) de glycérol, 18,0 g (0,3 mole) d'urée et 1,7 g de l'hydrate de p-toluène sulfate de zinc. On porte le mélange réactionnel à 145°C et on abaisse la pression à 50 mbar sous agitation mécanique pendant 1,25 heure. Pendant la réaction, l'ammoniac formé est éliminé par la pompe à vide 6. Après 1,25 heure, le mélange réactionnel brut est analysé par chromatographie en phase gazeuse sur colonne capillaire "Carbowax 20 M" (12 m) avec le tétraéthylène glycol comme étalon interne. 23,4 g (0,26 mole) de glycérol sont transformés en 30,1 g (mole) du carbonate de glycérol (soit un rendement molaire de 85 % par rapport au glycérol).

EXEMPLE 13

Une résine gel acide fort sous forme Na+ ("Bayer Lewatit" "VP OC 1800" marque déposée) prégonflée dans l'eau est permutée sous forme $Zn^{++}$ par percolation d'une solution $ZnSO_4$ 2 N (5 volumes pour 1 volume de résine). La résine permutée est rincée avec de l'eau déminéralisée jusqu'à l'élimination de $ZnSO_4$ libre (10 volumes pour 1 volume de résine). Elle est lavée à l'éthanol puis à l'oxyde de diéthyle. Elle est ensuite séchée sous vide d'une trompe à eau et stockée dans un dessicateur. Elle contient 1 meq.g de $Zn^{++}$ par ml.

Dans le réacteur 1 de la figure 1, on mélange 27,6 g (0,3 mole) de glycérol et 8 g de la résine ci-dessus préparée (soit sensiblement 8 ml). On porte le mélange réactionnel à 130°C et on abaisse la pression à 40 mbar sous agitation mécanique pendant 5 minutes pour l'élimination d'eau jusqu'à l'arrêt du dégagement de vapeurs d'eau. On élimine ainsi la quasi-totalité de l'eau de la résine. Ensuite on ajoute 18,0 g (0,3 mole) d'urée et on réalise la réaction pendant 5 heures à 130°C et à 40 mbar sous agitation mécanique. Pendant la réaction, l'ammoniac formé est éliminé par la pompe à vide 6. Après 5 heures, le mélange réactionnel brut est analysé par chromatographie en phase gazeuse sur colonne capillaire "Carbowax 20 M" (12 m) avec le tétraéthylène glycol comme étalon interne. 22,0 g (0,24 mole) de glycérol sont transformés en 28,2 g du carbonate de glycérol (soit un rendement molaire de 80 % par rapport au glycérol).

EXEMPLE 14

Une résine macroporeuse acide forte sous forme H+ ("Bayer K 2431" marque déposée) prégonflée dans l'eau est permutée sous forme $Na^+$ par percolation d'une solution NaOH 2 N (5 volumes pour 1 volume de résine). La résine permutée est rincée avec de l'eau déminéralisée jusqu'à l'obtention d'un effluent à pH neutre. Ensuite, elle est permutée sous forme métal bivalent $M^{++}$ par percolation d'une solution $MSO_4$ correspondant à 1 N (5 volumes pour 1 volume de résine). Le métal est différent selon les exemples : $Zn^{++}$ : ex 14; $Mn^{++}$ : ex 15; $Mg^{++}$ : ex 16 ; $Fe^{++}$ : ex 17 ; $Ni^{++}$ : ex 18 ; $Cd^{++}$ : ex 19. La résine permutée est rincée avec de l'eau déminéralisée jusqu'à l'élimination de $MSO_4$ libre (10 volumes pour 1 volume de résine). Elle est lavée à l'éthanol puis à l'oxyde de diéthyle. Elle est ensuite séchée sous vide d'une trompe à eau et stockée dans un dessicateur. Elle contient 0,5 meq.g de $M^{++}$ par ml.

La mise en oeuvre de l'exemple 14 est identique à celle de l'exemple 13 mais on change le catalyseur par la résine sous forme $Zn^{++}$ ci-dessus décrite (quantité : 8 g soit 8 ml). Après 5 heures, 22,1 g (0,24 mole) de glycérol sont transformés en 28,4 g du carbonate de glycérol (soit un rendement molaire de 81 % par rapport au glycérol).

EXEMPLES 15-19

La mise en oeuvre est identique à celle de l'exemple précédent, mais on change la température de la réaction pour l'ajuster à 145°C et le catalyseur est choisi parmi les résines dont la préparation est décrite à l'exemple précédent sous forme $Mn^{++}$, $Mg^{++}$, $Fe^{++}$, $Ni^{++}$, $Cd^{++}$. Après 2 heures de réaction, le mélange réactionnel brut est analysé par chromatographie en phase gazeuse sur colonne capillaire "Carbowax 20 M" (12 m) avec le tétraéthylène glycol comme étalon interne. Les rendements sont indiqués dans le tableau suivant.

| N° de l'exemple | Catalyseur | Rendement en 2 h (%) |
|---|---|---|
| 15 | $P-SO_3Mn$ | 63 |
| 16 | $P-SO_3Mg$ | 58 |
| 17 | $P-SO_3Fe$ | 61 |
| 18 | $P-SO_3Ni$ | 60 |
| 19 | $P-SO_3Cd$ | 57 |

EXEMPLE 20

Dans le réacteur 1 de la figure 1, on met 42,5 g de glycérol technique, c'est-à-dire disponible sur le marché (65 % de glycérol) soit 0,3 mole de glycérol. On porte le mélange réactionnel à 130°C et on abaisse la pression à 40 mbar sous agitation mécanique pendant 15 minutes pour l'élimination d'eau. On élimine ainsi la quasi-totalité de l'eau apportée par le glycérol. Ensuite, on ajoute 18,0 g (0,3 mole) d'urée et 1,7 g de sulfate de zinc (33 meq.g/mole de glycérol). On réalise la réaction pendant 5 heures à 130°C et 40 mbar sous agitation mécanique. Pendant la réaction, l'ammoniac formé est éliminé par la pompe à vide 6. Après 5 heures, le mélange réactionnel brut est analysé par chromatographie en phase gazeuse sur colonne capillaire "Carbowax 20 M" (12 m) avec le tétraéthylène glycol comme étalon interne. 21,8 g (0,237 mole) de glycérol sont transformés en 28,0 g du carbonate de glycérol (soit un rendement molaire de 79 % par rapport au glycérol). On constate donc que le procédé de l'invention donne un bon résultat en utilisant comme produit de départ un glycérol impur de type courant (glycérol dit technique).

EXEMPLE 21

Dans le réacteur 1 de la figure 1, on met 42,5 g de glycérol technique (65 % de glycérol) soit 0,3 mole de

glycérol. On porte le mélange réactionnel à 130°C et on abaisse la pression à 40 mbar sous agitation mécanique pendant 15 minutes pour l'élimination d'eau. Ensuite, on met 8 g de la résine préparée à l'exemple 13 sous forme $Zn^{++}$ et on porte le mélange réactionnel à 130°C et on abaisse la pression à 40 mbar sous agitation mécanique pendant 5 minutes pour l'élimination d'eau dans la résine. Après introduction de 18,0 g (0,3 mole) d'urée, on réalise la réaction pendant 5 heures à 130°C et à 40 mbar sous agitation mécanique. Pendant la réaction, l'ammoniac formé est éliminé par la pompe à vide 6. Après 5 heures de réaction, 20,7 g (0,225 mole) de glycérol sont transformés en 26,6 g de carbonate de glycérol (soit un rendement molaire de 75 % par rapport au glycérol).

## Revendications

1. Procédé de fabrication de carbonate de glycérol, **caractérisé en ce qu'**on fait réagir de l'urée et du glycérol à une température comprise entre 90° C et 220° C en présence d'un catalyseur portant des sites acides de Lewis associés à un ou des contre-ions anioniques d'hétéro-atome.

2. Procédé de fabrication de carbonate de glycérol selon la revendication 1, **caractérisé en ce qu'**on utilise comme catalyseur un sulfate métallique ou organométallique, se présentant sous forme de poudre solide, le cation métallique dudit sulfate étant choisi de façon que le sel présente une solubilité à froid dans l'eau supérieure à 5 g par litre.

3. Procédé de fabrication selon l'une des revendications 1 ou 2, **caractérisé en ce qu'**on utilise un sulfate métallique ou organornétallique ayant subi une calcination préalable.

4. Procédé de fabrication selon l'une des revendications 2 ou 3, **caractérisé en ce qu'**on utilise un sulfate métallique ou organométallique ou un mélange de sulfates métalliques ou organométalliques du groupe suivant : sulfate de manganèse, sulfate de zinc, sulfate de magnésium, sulfate de nickel, sulfate de fer, sulfate de cobalt, ou sulfate organométallique de zinc.

5. Procédé de fabrication selon l'une des revendications 2, 3 ou 4, **caractérisé en ce qu'**on ajoute le sulfate métallique ou organométallique dans une proportion pondérale par rapport au glycérol, comprise entre 1 % et 8 %.

6. Procédé de fabrication de carbonate de glycérol selon la revendication 1, **caractérisé en ce qu'**on utilise comme catalyseur un composé métallique supporté sous forme de solide macroporeux ou de gel, ledit composé comprenant une matrice polymérique organique supportant sur des groupements fonctionnels hétéro-organiques des cations métalliques dotés des sites acides de Lewis.

7. Procédé de fabrication selon la revendication 6, **caractérisé en ce qu'**on utilise un composé métallique supporté, obtenu à partir d'une matrice polymérique sulfonique par un échange d'ions apte à fixer sur ses groupements fonctionnels les cations métalliques dotés des sites acides de Lewis.

8. Procédé de fabrication selon l'une des revendications 6 ou 7, **caractérisé en ce qu'**on utilise un composé métallique supporté comprenant, d'une part, une matrice polymérique polystyrénique, d'autre part, un ou des cations du groupe suivant : $Zn^{++}$, $Mg^{++}$, $Mn^{++}$, $Fe^{++}$, $Ni^{++}$, $Cd^{++}$ associés à des groupements sulfonates de la matrice polystyrénique.

9. Procédé de fabrication selon l'une des revendications 6, 7 ou 8, **caractérisé en ce qu'**on ajoute le composé métallique supporté de façon que la quantité de cations métalliques par mole de glycérol soit comprise entre 10 et 100 milliéquivalent gramme.

10. Procédé de fabrication selon l'une des revendications 1 à 9, dans lequel on ajuste la pression du milieu réactionnel dans une plage comprise entre $10^3$ et $2.10^4$ pascals.

11. Procédé de fabrication selon la revendication 10, dans lequel le glycérol et le catalyseur sont d'abord mis en présence à la pression réduite précitée avec évacuation de la vapeur d'eau dégagée, l'urée étant ensuite ajoutée après élimination de l'eau libre du milieu réactionnel.

12. Procédé de fabrication selon l'une des revendications 1 à 11, dans lequel on ajuste les quantités d'urée et de glycérol mises en présence, de sorte que les proportions molaires relatives de ces composés soient comprises

entre 0,65 et 1,20 mole d'urée par mole de glycérol.

13. Procédé de fabrication selon l'une des revendications 1 à 12, dans lequel le glycérol est apporté sous forme de glycérine technique.

**Claims**

1. A method for preparing glycerol carbonate, wherein urea and glycerol are made to react at a temperature between 90°C and 220°C in the presence of a catalyst bearing Lewis acid sites associated with one or more hetero-atomic anionic counterions.

2. The method for preparing glycerol carbonate as claimed in claim 1, wherein a metallic or organometallic sulfate is used as a catalyst, in the form of a powdered solid, the metal cation of the said sulfate being chosen so that the salt has a cold water solubility greater than 5 g per litre.

3. The method for preparing glycerol carbonate as claimed in either of claims 1 or 2, wherein a metallic or organometallic sulfate is used which has been previously subjected to calcination.

4. The method for preparing glycerol carbonate as claimed in either of claims 2 or 3, wherein use is made of a metallic or organometallic sulfate or a mixture of metallic or organometallic sulfates from the following group: manganese sulfate, zinc sulfate, magnesium sulfate, nickel sulfate, iron sulfate, cobalt sulfate or an organometallic sulfate of zinc.

5. The method for preparing glycerol carbonate as claimed in one of claims 2, 3 or 4, wherein the metallic or organometallic sulfate is added in a weight proportion based on glycerol between 1 % and 8 %.

6. The method for preparing glycerol carbonate as claimed in claim 1, wherein a supported metallic compound is used as a catalyst in the form of a macroporous solid or of a gel, said compound comprising an organic polymeric matrix supporting, on hetero-organic functional groups, metal cations provided with Lewis acid sites.

7. The preparative method as claimed in claim 6, wherein a supported metallic compound is used, obtained from a sulfonic polymeric matrix by ion exchange, able to attach metal cations provided with Lewis acid sites onto its functional groups.

8. The preparative method as claimed in either claim 6 or 7, wherein a supported metallic compound is used comprising, on the one hand, a polystyrene polymeric matrix and, on the other hand, one or more cations of the following group: $Zn^{++}$, $Mg^{++}$, $Mn^{++}$, $Fe^{++}$, $Ni^{++}$, $Cd^{++}$, associated with the sulfonate groups of the polystyrene matrix.

9. The preparative method as claimed in one of claims 6, 7 or 8, wherein the supported metallic compound is added so that the quantity of metal cations per mole of glycerol is between and 100 milli-equivalent gram.

10. The preparative method as claimed in one of claims 1 to 9, wherein the pressure of the reaction medium is adjusted within a range between $10^3$ and $2.10^4$ pascals.

11. The preparative method as claimed in claim 10, wherein glycerol and the catalyst are first of all mixed at the aforementioned reduced pressure with evacuation of the water vapor evolved, urea being then added after free water has been eliminated from the reaction medium.

12. The preparative method as claimed in one of claims 1 to 11, wherein the quantities of urea and glycerol mixed are adjusted so that the relative molar proportions of these compounds lie between 0.65 and 1.20 mole of urea per mole of glycerol.

13. The preparative method as claimed in one of claims 1 to 12, wherein glycerol is added in the form of technical glycerine.

## Patentansprüche

1. Verfahren zur Herstellung von Glycerolcarbonat, **dadurch gekennzeichnet, dass** Harnstoff und Glycerol bei einer Temperatur zwischen 90°C und 220°C in Anwesenheit eines Katalysators reagieren gelassen werden, der Lewis-Säurestellen trägt, die mit (einem) anionischen Heteroatom-Gegenion(en) assoziiert sind.

2. Verfahren zur Herstellung von Glycerolcarbonat nach Anspruch 1, **dadurch gekennzeichnet, dass** als Katalysator ein metallisches oder metallorganisches Sulfat verwendet wird, das in Form eines festen Pulvers vorliegt, wobei das metallische Kation des genannten Sulfats so gewählt wird, dass das Salz eine Kaltlöslichkeit in Wasser von mehr als 5 g pro Liter hat.

3. Verfahren zur Herstellung nach Anspruch 1 oder 2, **dadurch gekennzeichnet, dass** ein metallisches oder metallorganisches Sulfat verwendet wird, das zuvor einer Kalzinierung unterzogen wurde.

4. Verfahren zur Herstellung nach Anspruch 2 oder 3, **dadurch gekennzeichnet, dass** ein metallisches oder metallorganisches Sulfat oder ein Gemisch aus metallischen oder metallorganischen Sulfaten der folgenden Gruppe verwendet wird: Mangansulfat, Zinksulfat, Magnesiumsulfat, Nickelsulfat, Eisensulfat, Cobaltsulfat oder metallorganisches Zinksulfat.

5. Verfahren zur Herstellung nach Anspruch 2, 3 oder 4, **dadurch gekennzeichnet, dass** das metallische oder metallorganische Sulfat in einem Gewichtsanteil von 1 bis 8% in Bezug auf das Glycerol zugegeben wird.

6. Verfahren zur Herstellung von Glycerolcarbonat nach Anspruch 1, **dadurch gekennzeichnet, dass** als Katalysator eine metallische Verbindung verwendet wird, die in der Form eines makroporösen Feststoffs oder von Gel getragen wird, wobei die genannte Verbindung eine organische Polymermatrix umfasst, die auf heteroorganischen Funktionsgruppen metallische Kationen trägt, die mit Lewis-Säurestellen dotiert sind.

7. Verfahren zur Herstellung nach Anspruch 6, **dadurch gekennzeichnet, dass** eine getragene metallische Verbindung verwendet wird, die von einer Sulfonpolymermatrix durch einen Ionenaustausch erhalten wurde, mit dem die mit Lewis-Säurestellen dotierten metallischen Kationen auf ihren Funktionsgruppen fixiert werden können.

8. Verfahren zur Herstellung nach Anspruch 6 oder 7, **dadurch gekennzeichnet, dass** eine metallische getragene Verbindung verwendet wird, die einerseits eine Polystyrol-Polymermatrix und andererseits (ein) Kation(en) der folgenden Gruppe enthalten: $Zn^{++}$, $Mg^{++}$, $Mn^{++}$, $Fe^{++}$, $Cd^{++}$, die mit Sulfonatgruppen der Polystyrolmatrix assoziiert sind.

9. Verfahren zur Herstellung nach Anspruch 6, 7 oder 8, **dadurch gekennzeichnet, dass** die metallische getragene Verbindung so zugegeben wird, dass die Menge der metallischen Kationen pro Mol Glycerol zwischen 10 und 100 Milliäquivalent-Gramm beträgt.

10. Verfahren zur Herstellung nach einem der Ansprüche 1 bis 9, bei dem der Druck der Reaktionsumgebung im Bereich zwischen $10^3$ und $2 \times 10^4$ Pascal eingestellt wird.

11. Verfahren zur Herstellung nach Anspruch 10, bei dem zunächst das Glycerol und der Katalysator bei dem oben angegebenen reduzierten Druck unter Evakuierung des freigesetzten Wasserdampfes eingeleitet werden, wobei der Harnstoff anschließend nach der Eliminierung des freien Wassers der Reaktionsumgebung zugegeben wird.

12. Verfahren zur Herstellung nach einem der Ansprüche 1 bis 11, bei dem die eingeleiteten Harnstoff- und Glycerolmengen so eingestellt werden, dass die relativen Molanteile dieser Zusammensetzungen zwischen 0,65 und 1,20 Mol Harnstoff pro Mol Glycerol betragen.

13. Verfahren zur Herstellung nach einem der Ansprüche 1 bis 12, bei dem das Glycerol in eine glycerintechnische Form gebracht wird.

Fig 1